# EUROPÄISCHE PATENTSCHRIFT

(11) **EP 0 799 175 B1**
(45) Veröffentlichungstag und Bekanntmachung des Hinweises auf die Patenterteilung: **14.10.1998**
(21) Anmeldenummer: 95943187.5
(22) Anmeldetag: 22.12.1995
(51) Int. Cl.: C07C 49/217, C07C 49/82, C07C 255/25, C07C 255/36, C07D 311/08, C25D 3/22

(54) **SALZE AROMATISCHER HYDROXYLVERBINDUNGEN UND DEREN VERWENDUNG ALS GLANZBILDNER**
SALTS OF AROMATIC HYDROXYL COMPOUNDS AND THEIR USE AS BRIGHTENERS
SELS DE COMPOSES HYDROXYLES AROMATIQUES ET LEUR UTILISATION COMME LUSTRANTS

(30) Priorität: 23.12.1994 DE 4446329
(43) Veröffentlichungstag der Anmeldung: 08.10.1997
(73) Patentinhaber: BASF AKTIENGESELLSCHAFT, 67056 Ludwigshafen (DE)
(72) Erfinder: WEHLAGE, Thomas, D-67346 Speyer (DE); SCHRÖDER, Ulrich, D-67227 Frankenthal (DE); OFTRING, Alfred, D-67098 Bad Dürkheim (DE)
(74) Vertreter: Isenbruck, Günter, Dr.
(86) Internationale Anmeldenummer: EP9505090
(87) Internationale Veröffentlichungsnummer: WO9620152

(56) Entgegenhaltungen:
- EP-A- 0 069 536
- EP-A- 0 376 288
- WO-A-94/19317
- US-A- 3 755 450
- US-A- 3 773 630
- US-A- 4 218 292
- PATENT ABSTRACTS OF JAPAN vol. 014 no. 043 (C-0681) ,26.Januar 1990 & JP,A,01 275552 (SUNTORY LTD) 6.November 1989,

## Beschreibung

### I. Gegenstand der Erfindung

Die vorliegende Erfindung betrifft Salze aromatischer Hydroxylverbindungen sowie deren Verwendung als Glanzbildner. Weiterhin betrifft die Erfindung saure galvanische Bäder sowie ein Verfahren zur galvanischen Beschichtung von Formteilen.

### II. Hintergrund der Erfindung

Bei der galvanischen Abscheidung von Metallen bzw. Legierungen, insbesondere von Zink oder Zinn bzw. Zink- oder Zinnlegierungen, auf metallischen Substraten, wie Metallformteilen oder metallisierten Kunststofformteilen, aus wäßrig-saurer Lösung wird häufig ein glänzender Überzug angestrebt, um dem galvanisierten Gegenstand ein vorteilhaftes dekoratives Aussehen zu verleihen. Somit ist oft neben dem korrosionsverhindernden bzw. einem die mechanischen Eigenschaften des Formteils verbessernden Effekt auch ein dekorativer Effekt erwünscht. Um die gewünschten Wirkungen zu erzielen, ist es unerläßlich, daß die galvanischen Bäder bestimmte Hilfsmittel enthalten, weil andernfalls die sich aus saurer Lösung abscheidenden Metallüberzüge meistens matt und häufig auch noch unregelmäßig anfallen. Eine Gruppe solcher Hilfsmittel für saure galvanische Bäder sind beispielsweise Leitsalze, die zur Verbesserung der Leitfähigkeit der Bäder eingesetzt werden. Eine andere Gruppe von Hilfsmittel sind die sog. Glanzbildner.

In der US-PS 3 694 330 (Reissue 27 999) werden saure galvanische Zinkbäder, die Ammoniumsalze und als Glanzbildner aromatische Carbonylverbindungen enthalten, offenbart. Als letztere werden aromatische Carbonsäuren sowie aromatische Aldehyde und Ketone, die teilweise Hydroxylgruppen aufweisen, genannt. Explizit angeführt sind u.a. Zimtsäure, Zimtaldehyd, Benzoesäure, Benzalaceton und Benzoylessigsäureethylester sowie m-Hydroxybenzaldehyd.

In der US-PS 4 422 908 werden saure galvanische Zinkbäder, die Sulfämationen und als Glanzbildner aromatische Carbonylverbindungen enthalten, beschrieben. Es werden aromatische Aldehyde und Ketone angeführt, darunter als bevorzugte Substanz Benzalaceton.

In der DE-A-29 48 261 werden saure galvanische Zinkbäder, die gegebenenfalls Ammoniumsalze enthalten, beschrieben, die als Glanzbildner aromatische, carbonylhaltige Verbindungen, z.B. aromatische Aldehyde, die teilweise Hydroxylgruppen aufweisen, wie o-Chlorbenzaldehyd, p-Chlorbenzaldehyd, o-Hydroxybenzaldehyd, Aminobenzaldehyd, Veratrumaldehyd, Benzylidenaceton (Benzalaceton), Cumarin, 3,4,5,6-Tetrahydrobenzaldehyd, Acetophenon, Propiophenon, Furfurylidenaceton, 3-Methoxybenzalaceton, Benzaldehyd, Vanillin, Hydroxybenzaldehyd, Anisaldehyd, Benzoesäure, Natriumbenzoat, Natriumsalicylat oder 3-Pyridincarbonsäure (Nikotinsäure) enthalten. Zur Verbesserung der Eigenschaften der Galvanisierbäder wird hierbei die Verwendung von polymeren, schwefelhaltigen Verbindungen einer näher definierten allgemeinen Formel beschrieben.

Der wichtigste Vertreter solcher Glanzbildner für saure Zinkbäder ist Benzylidenaceton (Benzalaceton). Wegen seines merklichen Dampfdrucks kann sich Benzylidenaceton aus galvanischen Bädern verflüchtigen, was zu Substanzverlusten führt. Hinzu kommt, daß diese Verbindungen sowie deren Homologen bei vielen Personen, die damit umgehen, allergische Reaktionen, wie Hautstörungen und Juckreiz, hervorrufen. Zudem ist Benzalaceton nicht wasserlöslich und muß mit Hilfe von zumeist toxischen Lösungsmitteln solubilisiert werden. Diese Lösungsmittel weisen häufig einen niedrigen Flammpunkt auf, weshalb zusätzliche Sicherheitsmaßnahmen getroffen werden müssen.

In der DE-A-41 19 341 (2) wird empfohlen, Glanzbildner auf Basis von Benzylidenaceton in wäßrig-sauren, galvanischen Bädern zur Abscheidung von Zink oder Zinklegierungen durch bestimmte andere Substanzen zu ersetzen, die die Wirksamkeit der bekannten Glanzbildner erreichen, jedoch nicht zu gesundheitlichen Beeinträchtigungen bei Personen führen, die diese Substanzen handhaben. Die dort offenbarten Verbindungen weisen jedoch die übrigen, oben angegebenen Nachteile auf.

Die nachstehend aufgeführten Verbindungen gehören zur Gruppe der Hydroxylgruppen aufweisenden Aromaten.

In der EP-A1-0 376 288 sind Naphthylenderivate beschrieben, die am aromatischen Kern Hydroxylgruppen und eine über eine Ethylengruppe gebundene Carbonylgruppe enthalten. Die Verbindungen werden als Arzneistoffe verwendet. Eine Verwendung in galvanischen Bädern ist nicht beschrieben.

In JP-A-1 275 552 sind Kaffeesäurederivate beschrieben, die am ethylenisch ungesättigten Rest eine Cyanogruppe aufweisen. Sie werden als Arzneimittel verwendet. Eine Verwendung in galvanischen Bädern ist nicht beschrieben.

In der EP-A1-0 069 536 sind Phlorophenonderivate beschrieben, in denen an einen subsituierten Phenylrest ein Acylrest gebunden ist, der einen verzweigten oder unverzweigten Alkyl-, Cycloalkyl- oder Aralkylrest aufweist. Die Verbindungen werden als Arzneimittel eingesetzt. Eine Verwendung in galvanischen Bädern ist nicht beschrieben.

In der WO 94/19317 ist ein Verfahren zur Herstellung von o-hydroxisubstituierten aromatischen Nitrilen beschrieben. Die aromatischen Nitrile können eine Reihe von Substituenten aufweisen und als Salze zweiwertiger Metalle, wie Magnesium, vorliegen. Insbesondere wird 2-Cyanophenol aufgeführt. Die Verbindungen können zur Herstellung von Landwirtschaftschemikalien verwendet werden. Eine Verwendung in galvanischen Bädern ist nicht beschrieben.

In der US 3,755,450 sind 2-Hydroxy-4-alkoxybenzophenone beschrieben, die in Kombination mit Magnesium oder Zinksalzen von Fettsäuren in frei fließenden UV-Absorberzusammensetzungen eingesetzt werden. Eine Verwendung in galvanischen Bädern ist nicht beschrieben.

Der vorliegenden Erfindung lag daher die Aufgabe zugrunde, Verbindungen als Glanzbildner in wäßrig-sauren galvanischen Bädern bereitzustellen, die die Wirksamkeit der bekannten Glanzbildner, insbesondere des Benzylidenacetons, erreichen und dabei einen geringeren Dampfdruck als Einzelsubstanz und in den galvanischen Bädern aufweisen, um Substanzverluste zu vermeiden. Die neuen Verbindungen sollten weiterhin wasserlöslich sein, so daß auf organische Lösungsmittel verzichtet und die Konzentration von Solubilisatoren in den galvanischen Bädern reduziert werden kann.

### III. Beschreibung der Erfindung

Diese Aufgabe wird erfindungsgemäß durch aromatische Verbindungen der Formel I gelöst, 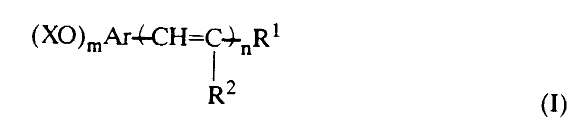 worin
- m: für eine ganze Zahl ≥ 1 steht;
- n: 0 oder 1 bedeutet;
- Ar: einen gegebenenfalls mit einem oder mehreren Resten, die gleich oder verschieden sein können und ausgewählt sind aus
C₁-C₈-Alkyl-,
C₁-C₈-Alkoxy-,
C₁-C₄-Alkoxycarbonyl-,
Halogen-,
Phenyl- oder
Benzylresten substituierten Phenylen- oder Naphthylenrest bedeutet;
- R¹: einen Cyano-, oder einen Acylrest der Formel -COR³ bedeutet, worin
R³ für einen gegebenenfalls mit einem oder mehreren Resten, die gleich oder verschieden sein können und ausgewählt sind aus
C₁-C₄-Alkoxycarbonyl-,
C₁-C₄-Alkoxy-
Carbonyl- oder
Cyanoresten substituierten C₁-C₈-Alkyl-, C₂-C₈-Alkenyl- oder C₂-C₈-Alkinylrest; für einen gegebenenfalls mit einem oder mehreren Resten, die gleich oder verschieden sein können und ausgewählt sind aus
C₁-C₄-Alkyl-,
C₁-C₄-Alkoxy-,
C₁-C₄-Alkoxycarbonyl-,
Halogen-,
Hydroxyl- oder
Phenylresten substituierten Phenyl- oder Benzylrest;
für einen C₅-C₈-Cycloalkylrest;
für einen Hydroxylrest;
für einen C₁-C₄-Alkoxyrest; oder
für ein Wasserstoffatom steht;
- R²: ein Wasserstoffatom,
einen C₁-C₈-Alkyl-,
Phenyl-,
Benzyl-,
Cyano- oder
Hydroxlyrest, oder
einen Rest der Formel
-COR⁴,
-COOR⁴,
-COCH₂COOR⁴,
-OR⁴ oder
-CONR⁴R⁵,
worin
R⁴ und R⁵, die gleich oder verschieden sein können,
für ein Wasserstoffatom,
einen C₁-C₈-Alkyl-,
Phenyl- oder
Benzylrest stehen,
und
- X: ein Alkalimetall-,
Erdalkalimetallatom oder
einen Ammoniumrest bedeutet,
mit der Maßgabe, daß das Natriumsalz von Vanillidenaceton sowie das Natriumsalz von 5-Bromvanillindenaceton ausgenommen sind.

Weiterhin wird die Aufgabe durch Verbindungen der Formel II gelöst, 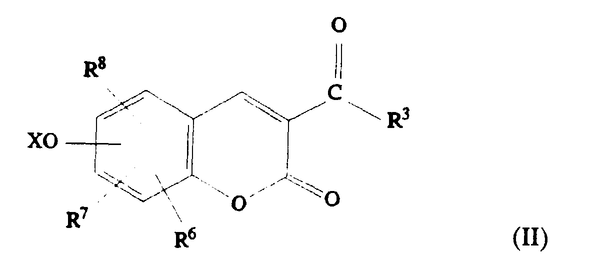 worin
R⁶, R⁷ und R⁸, die gleich oder verschieden sein können,
   ein Wasserstoffatom,
   einen C₁-C₈-Alkyl-,
   C₁-C₈-Alkoxy-,
   C₁-C₄-Alkoxycarbonyl-,
   Halogen-,
   Phenyl- oder
   Benzylrest, oder
   einen Rest der Formel -OX bedeuten, und
R³ und X die bei den Verbindungen der Formel I angegebenen Bedeutungen besitzen.

Die Verbindungen der Formel II sind als innere Ester (Lactone der entsprechenden Cumarinsäurederivate) von Verbindungen der Formel I aufzufassen.

Die Verbindungen der Formel I bzw. II stellen Salze von aromatischen Hydroxylverbindungen dar und sind überraschenderweise ausgezeichnet wasserlöslich. Weiterhin besitzen die Verbindungen sowohl in reiner Form als auch in wäßriger Lösung in galvanischen Bädern einen geringeren Dampfdruck als die entsprechenden Neutralverbindungen des Standes der Technik.

Bei den Verbindungen der Formel I und II seien genannt:
- als C₁-C₈-Alkylreste:: Methyl, Ethyl, n-Propyl, iso-Propyl, n-Butyl, iso-Butyl, t-Butyl, n-Pentyl, iso-Pentyl, n-Hexyl, iso-Hexyl, n-Heptyl, iso-Heptyl, n-Octyl, iso-Octyl;
- als C₁-C₈-Alkoxyreste:: Methoxy, Ethyloxy, n-Propyloxy, iso-Propyloxy, n-Butyloxy, iso-Butyloxy, t-Butyloxy, n-Pentyloxy, iso-Pentyloxy, n-Hexyloxy, iso-Hexyloxy, n-Heptyloxy, iso-Heptyloxy, n-Octyloxy, iso-Octyloxy;
- als C₁-C₄-Alkoxycarbonylreste:: Methoxycarbonyl, Ethyloxycarbonyl, n-Propyloxycarbonyl, iso-Propyloxycarbonyl, n-Butyloxycarbonyl, iso-Butyloxycarbonyl, tert.-Butyloxycarbonyl;
- als Halogenreste:: F, Cl, Br, I;
- als C₁-C₄-Alkoxyreste:: Methoxy, Ethyloxy, n-Propyloxy, iso-Propyloxy, n-Butyloxy, iso-Butyloxy, tert.-Butyloxy;
- als C₂-C₈-Alkenylreste:: Ethenyl, n-Propenyl, iso-Propenyl, n-Butenyl, n-Pentenyl, n-Hexenyl, n-Heptenyl, n-Octenyl;
- als C₂-C₈-Alkinylreste:: Ethinyl, Propinyl, n-Butinyl, n-Pentinyl, n-Hexinyl, n-Heptinyl, n-Octinyl;
- als C₁-C₄-Alkylreste:: Methyl, Ethyl, n-Propyl, iso-Propyl, n-Butyl, iso-Butyl, tert.-Butyl;
- als Alkalimetallatome:: Li, Na, K, Rb, Cs;
- als Erdalkalimetallatome:: Mg, Ca, Sr, Ba.

Bei den Verbindungen der Formel I, in denen n für 1 steht, bedeuten Ar bevorzugt einen gegebenenfalls mit einem oder mehreren, insbesondere mit einem oder zwei Resten, die gleich oder verschieden sein können und ausgewählt sind aus
C₁-C₄-Alkyl-, insbesondere Methyl- oder Ethyl-;
C₁-C₄-Alkoxy-, insbesondere Methoxy- oder Ethoxyresten; und
Br oder Cl;
substituierten Phenylenrest;
und/oder
R¹ einen Cyano-, oder
   einen Acylrest der Formel -COR³, worin
   R³ für einen gegebenenfalls mit
   einem oder mehreren C₁-C₄-Alkoxy-, insbesondere Methoxy- oder Ethoxyresten, die gleich oder verschieden sein können,
   substituierten C₁-C₄-Alkyl-, insbesondere Methyl- oder Ethylrest;
   einen gegebenenfalls mit einem oder mehreren Resten, die gleich oder verschieden sein können und ausgewählt sind aus
   C₁-C₄-Alkyl-, insbesondere Methyl- oder Ethyl-;
   C₁-C₄-Alkoxy-, insbesondere Methoxy- oder Ethoxy-;
   Hydroxylresten; oder
   Br oder Cl;
   substituierten Phenyl- oder Benzylrest;
   einen Hydroxylrest; oder
   einen C₁-C₄-Alkoxyrest steht; und/oder
R² bevorzugt ein Wasserstoffatom; oder einen Rest der Formel
   -COR⁴;
   -COOR⁴;
   -COCH₂COOR⁴; oder
   -CONR⁴R⁵;
   worin
   R⁴ und R⁵, die gleich oder verschieden sein können, für
   ein Wasserstoffatom;
   einen C₁-C₄-Alkyl-, insbesondere Methyl- oder Ethyl-;
   einen Phenyl-; oder
   einen Benzylrest stehen.

Bei den Verbindungen der Formel I, in denen n für 0 steht, bedeuten
- Ar: bevorzugt einen gegebenenfalls mit einem oder mehreren, insbesondere mit einem oder zwei Resten, die gleich oder verschieden sein können und ausgewählt sind aus
C₁-C₄-Alkyl-, insbesondere Methyl- oder Ethyl-;
C₁-C₄-Alkoxy-, insbesondere Methoxy- oder Ethoxy-; Halogen-, insbesondere Br oder Cl; und Hydroxylresten; substituierten Phenylen- oder Naphthylenrest;
und/oder
- R¹: bevorzugt einen Acylrest der Formel -COR³,
worin
R³ für einen gegebenenfalls mit einem oder mehreren C₁-C₄-Alkoxy-, insbesondere Methoxy- oder Ethoxyresten, die gleich oder verschieden sein können, substituierten C₁-C₄-Alkylrest; für einen gegebenenfalls mit einem oder mehreren Resten, die gleich oder verschieden sein können und ausgewählt sind unter
C₁-C₄-Alkyl-, insbesondere Methyl- oder Ethyl-;
C₁-C₄-Alkoxy-, insbesondere Methoxy- oder Ethoxy-; und
Br oder Cl
substituierten Benzyl- oder Phenylrest; oder
für ein Wasserstoffatom steht. Bei den Verbindungen der Formel II bedeuten
- R3: bevorzugt einen gegebenenfalls mit einem oder mehreren C₁-C₄-Alkoxy-, insbesondere Methoxy- oder Ethoxyresten, die gleich oder verschieden sein können, substituierten C₁-C₄-Alkyl-, insbesondere Methyl- oder Ethylrest;
einen gegebenenfalls mit einem oder mehreren Resten, die gleich oder verschieden sein können und ausgewählt sind aus
C₁-C₄-Alkyl-, insbesondere Methyl- oder Ethyl-;
C₁-C₄-Alkoxy-, insbesondere Methoxy- oder Ethoxy-;
Hydroxylresten; oder
Br oder Cl;
substituierten Phenyl- oder Benzylrest;
einen Hydroxylrest; oder
einen C₁-C₄-Alkoxyrest; und/oder die Reste R⁶, R⁷ und R⁸, die gleich oder verschieden sein können, bevorzugt
ein Wasserstoffatom,
einen C₁-C₄-Alkoxy-, insbesondere einen Methoxy- oder Ethoxyrest, oder
einen Rest der Formel -OX, worin X eine der bei den Verbindungen der Formel I angegebenen Bedeutungen besitzt.

In den Verbindungen der Formel I oder II steht X bevorzugt für ein Alkalimetallatom, insbesondere für Na oder K.

Bevorzugt sind Verbindungen der Formel I, worin
- n: für 1 steht;
- m: für 1 steht;
- Ar: einen gegebenenfalls mit einem Methoxy- oder Ethoxyrest substituierten Phenylenrest bedeutet;
- R¹: einen Cyano- oder
einen Acylrest der Formel -COR³ bedeutet, worin
R³ für einen
Methyl- oder Ethyl-;
Methoxy- oder Ethoxyrest steht; und
- R²: ein Wasserstoffatom,
einen Rest der Formel
-COR⁴;
-COOR⁴;
-CONHR⁵; oder
-CONR⁴R⁵ bedeutet,
worin
R⁴ für einen Methyl- oder Ethylrest steht; und
R⁵ für
ein Wasserstoffatom;
einen Methyl- oder Ethyl-;
Phenyl-; oder
Benzylrest steht;
und
- X: Na oder K bedeutet.

Bevorzugt sind auch Verbindungen der Formel I, worin
- n: für 0 steht;
- m: für 1 steht;
- Ar: einen gegebenenfalls mit einem
Methyl- oder Ethyl-;
Methoxy- oder Ethoxy-; substituierten Phenylen- oder Naphthylenrest bedeutet;
- R¹: einen Acylrest der Formel -COR³ bedeutet,
worin
R³ für ein Wasserstoffatom oder
einen Phenylrest steht; und
- X: Na oder K bedeutet.

Weiterhin bevorzugt sind Verbindungen der Formel II, worin
- R³: einen Methoxy- oder Ethoxyrest bedeutet;
einer der Reste R⁶, R⁷ und R⁸ ein Wasserstoffatom; oder einen Methoxy- oder Ethoxyrest,
und die anderen beiden Reste jeweils ein Wasserstoffatom bedeuten; und
- X: Na oder K bedeutet.

Die zur Herstellung der Verbindungen der Formel I (n=1) als Ausgangssubstanzen verwendeten Hydroxybenzylidenderivate und Hydroxynaphthylidenderivate sind beispielsweise durch eine Aldolkondensation bzw. Knoevenagel-Kondensation der entsprechend substituierten Hydroxybenzaldehydbzw. Hydroxynaphthaldehydverbindungen mit den entsprechend substituierten Alkylnitril- oder Alkylcarbonylverbindungen, oder den entsprechend substituierten Acylessigsäure- oder Cyanoessigsäurederivaten sowie deren Estern und Amiden erhältlich. Bei der Synthese der Benzylidenacetonderivate fallen dabei sowohl die E- als auch die Z-Derivate an. Entsprechende Reaktionen sind beispielsweise von Zoeller und Sumner in J. Org. Chem. **55** (1990), S. 319-324, beschrieben worden.

Die zur Herstellung von Verbindungen der Formel I (n = 0) als Ausgangssubstanzen verwendeten o-Hydroxybenzophenone lassen sich beispielsweise durch eine Fries-Umlagerung auf übliche Weise aus den entsprechend substituierten Arylestern herstellen. Entsprechende Reaktionen sind beispielsweise beschrieben in H. Henecka, Houben-Weyl, Methoden der organischen Chemie, Bd. 7/2a, S. 379 ff., Thieme-Verlag, Stuttgart 1973.

Die zur Herstellung der Verbindungen der Formel II als Ausgangssubstanzen verwendeten Hydroxycumarinderivate sind durch eine Knoevenagel-Kondensation von entsprechend substituierten Hydroxysalicylaldehydderivaten mit entsprechend substituierten Acetessigsäurederivaten erhältlich. Entsprechende Reaktionen sind von van den Goorbergh et al. in Synthesis (1984), S. 859-860, beschrieben worden.

Die erfindungsgemäßen Salze der Verbindungen der Formeln I und II lassen sich durch Umsetzung der Hydroxylderivate beispielsweise mit den entsprechenden Alkalimetall-, Erdalkalimetall- oder Ammoniumhydroxiden herstellen. Die Herstellung des Natriumsalzes von Vanillidenaceton und des Natriumsalzes von 5-Bromvanillidenaceton wird beispielsweise von Glaser und Tramer in J. Prakt. Chem. **116** (1927), S. 331-346, beschrieben.

Einen weiteren Gegenstand der vorliegenden Erfindung stellt ein saures galvanisches Bad zur elektrolytischen Abscheidung von metallischen Schichten auf Formteilen dar, das ein oder mehrere Metallsalz(e) einen oder mehrere Glanzbildner, gegebenenfalls ein oder mehrere Leitsalz(e) und gegebenenfalls einen oder mehrere Hilfsglanzbildner umfaßt, wobei das Bad als Glanzbildner mindestens eine der oben genannten Verbindungen der Formeln I oder II umfaßt, das Natriumsalz von Vanillidenaceton und/oder das Natriumsalz von 5-Bromvanillidenaceton umfaßt.

Als Metallsalz(e) sind Zink- und Zinnsalze bevorzugt.

Die Verbindungen der Formeln I oder II werden in den erfindungsgemäßen sauren galvanischen Bädern zweckmäßigerweise in einer Menge von 0,01-3 g/l, bevorzugt von 0,05-1,5 g/l, und besonders bevorzugt von 0,1-0,7 g/l, eingesetzt.

Es ist auch möglich, Mischungen von Verbindungen der Formeln I oder II als Glanzbildner einzusetzen sowie die Glanzbildner der Formeln I oder II zusammen mit anderen bekannten Glanzbildnern zu verwenden.

Als zusätzliche Glanzbildner können mit vorteilhafter Wirkung Benzoate, bevorzugt Alkalimetall- oder Ammoniumbenzoate, insbesondere Natriumbenzoat, in Konzentrationen von 0,1-8 g/l, vorzugsweise von 14 g/l, eingesetzt werden.

Die erfindungsgemäßen wäßrig-sauren galvanischen Bäder weisen bezüglich der übrigen Komponenten die üblichen Zusammensetzungen auf. Sie enthalten beispielsweise 50-150 g/l Zinkchlorid oder die äquivalente Menge Zinksulfat. Sofern Legierungen des Zinks, beispielsweise mit Kobalt und/oder Nickel und/oder Eisen auf metallischen Formteilen abgeschieden werden sollen, enthalten die Bäder zusätzlich in der Regel von 1-30 g/l Kobalt- und/oder Nickel- und/oder Eisensulfat oder die äquivalente Menge eines anderen, wasserlöslichen Kobalt- und/oder Nickel- und/oder Eisensalzes. Weiterhin ist es möglich, die entsprechenden Zinnsalze in äquivalenten Mengen zu verwenden.

Ein weiterer üblicher Bestandteil der Bäder sind Leitsalze, insbesondere Natrium-, Kalium- und Ammoniumsalze. Ein geeignetes Leitsalz ist Kaliumchlorid, das üblicherweise in einer Konzentration von 100-250 g/l in einem erfindungsgemäßen galvanischen Bad enthalten ist. Andere Leitsalze sind beispielsweise Ammoniumchlorid oder Natriumchlorid, die üblicherweise in einer Konzentration von 10-150 g/l eingesetzt werden.

Ein weiterer üblicher Bestandteil der erfindungsgemäßen wäßrig-sauren galvanischen Bäder sind Tenside bzw. Netzmittel, insbesondere nichtionische und anionische Tenside, die als Hilfsglanzbildner wirken. Geeignete nichtionische Tenside sind beispielsweise aus der GB-PS-1 149 106 bekannt. Hierbei handelt es sich um Additionsprodukte von Ethylenoxid an Fettalkohole, z.B. an C₈-C₁₈-Alkohole, oder um Additionsprodukte von Ethylenoxid an Phenol- bzw. Alkylphenole, insbesondere an Nonylphenol. Pro Mol Alkohol bzw. Phenol werden dabei in der Regel 5-100 Mol Ethylenoxid addiert. Weiterhin können polyoxyalkylierte Naphthole zugesetzt werden.

Zu weiteren brauchbaren nichtionischen Tensiden zählen Poly(alkylenimine). Poly(alkylenimine) können als solche verwendet werden, oder sie können mit einem zyklischen Carbonat umgesetzt werden, das aus Kohlenstoff-, Wasserstoff- und Sauerstoffatomen besteht. Eine Beschreibung für die Herstellung solcher Verbindungen ist in der US-PS-2 824 857 offenbart.

Geeignete anionische Tenside sind beispielsweise in der US-PS-3 787 296 offenbart. Es handelt sich hierbei hauptsächlich um sulfatierte Polyether, die beispielsweise durch Addition von Ethylenoxid an Fettalkohole, Fettamine, Amide von C₆-C₁₀-Carbonsäuren und längerkettige Fettsäuren sowie eine jeweils nachfolgende Sulfonierung erhältlich sind. Auch Sulfonate von Polyalkylenoxiden oder Blockcopolymerisaten aus Ethylenoxid und Propylenoxid werden als anionische Tenside verwendet.

Eine weitere Gruppe von anionischen Tensiden ist in der EP-B-115 020 offenbart. Es werden sulfonierte und sulfatierte Alkylphenolethoxylate der allgemeinen Formel 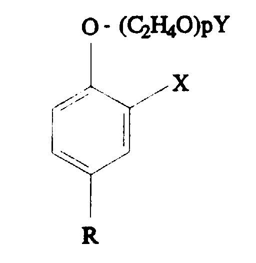 beschrieben, in der R einen C₄-C₂₀-Alkylrest, X und Y einen Rest der Formeln -SO₃H oder -SO₃Me, wobei Me für einen Ammoniumrest, ein Alkalimetall- oder ein äquivalentes Erdalkalimetall- oder Zinkatom steht, und p eine Zahl von 5-50 bedeuten.

Außer solchen Verbindungen kommen als anionische Tenside auch die sulfonierten und sulfatierten Produkte in Betracht, deren Polyetherkette 1-25 Propylenoxid- oder Butylenoxideinheiten enthält.

Die DE-C-38 39 824 offenbart anionaktive Additive auf Basis von einund mehrbasigen Ethersulfonsäuren. Diese werden durch einzelne oder kombinierte Sulfopropylierung und/oder Sulfobutylierung von folgenden hydroxylhaltigen Verbindungen hergestellt:
a) Blockpolymerisate von Ethylenoxid und/oder Glycidol mit Propylenoxid und/oder Butylenoxid;
b) ein- oder mehrwertige, gesättigte oder ungesättigte aliphatische Alkohole sowie ein- oder mehrwertige, alkylierte oder nichtalkylierte Phenole oder Naphthole, einschließlich ihrer Alkoxylate.

Der Vorteil der sulfonierten und sulfatierten Alkylphenolalkoxylate liegt darin, daß sie einen außerordentlich hohen Trübungspunkt aufweisen, so daß die elektrolytische Abscheidung des Metalls, insbesondere des Zinks, nicht nur im Temperaturbereich von 20-30°C, sondern auch bei Temperaturen von 30-90°C, vorzugsweise im Bereich von 40-50°C, vorgenommen werden kann.

Zu weiteren geeigneten Tensiden zählen Phenol-Formaldehyd-Kondensationsprodukte oder Naphthalinsulfonsäure/Formaldehyd-Kondensationsprodukte.

Neben den genannten Tensiden stellen auch Polyethylenglycole mit Molekulargewichten von 200-1000 g/mol geeignete Hilfsglanzbildner dar.

Die Tenside bzw. andere Hilfsglanzbildner werden in den erfindungsgemäßen wäßrig-sauren galvanischen Bädern üblicherweise in Mengen von 1-20 g/l, vorzugsweise von 2-15 g/l, eingesetzt. Weiterhin ist es möglich, eine Mischung von mehreren Tensiden bzw. Hilfsglanzbildnern zu verwenden.

Der pH-Wert der erfindungsgemäßen wäßrig-sauren galvanischen Bäder liegt in der Regel im Bereich von 3-7, vorzugsweise von 4-5. Er wird beispielsweise durch Zugabe von Säuren, z.B. üblichen Mineralsäuren wie Schwefelsäure oder Salzsäure, eingestellt.

Ein weiterer Gegenstand der vorliegenden Erfindung ist ein Verfahren zur galvanischen Beschichtung von Formteilen, bei dem man
1. ein Formteil mit einem erfindungsgemäßen sauren galvanischen Bad in Kontakt bringt, und
2. eine Galvanisierung durchführt.

Durch das erfindungsgemäße Verfahren werden beispielsweise Formteile aus Metallen, hauptsächlich aus Eisen oder Stahl, galvanisiert, um sie gegen Korrosion zu schützen und ihnen gleichzeitig einen hohen Glanz zu verleihen. Die hierzu verwendeten erfindungsgemäßen sauren galvanischen Bäder liefern über den gesamten, technisch in Betracht kommenden Stromdichtebereich hochglänzende und duktile Metallüberzüge, beispielsweise Zinküberzüge, die in der Qualität den Überzügen entsprechen oder sogar überlegen sind, die unter Verwendung von Benzylidenaceton nach dem Stand der Technik erhältlich sind.

Im folgenden sind Synthesebeispiele für Verbindungen der Formeln I und II sowie Anwendungsbeispiele für erfindungsgemäße saure galvanische Bäder aufgeführt.

### IV. Beispiele

Die Gegenstände der vorliegenden Erfindung werden durch die nachfolgenden Beispiele erläutert, in denen weitere bevorzugte Einzelmerkmale der Erfindung beschrieben sind.

### 1. Synthesebeispiele

Nachfolgend sind Synthesebeispiele für einige der erfindungsgemäßen Verbindungen der Formeln I bzw. II aufgeführt.

### Beispiel 1

Herstellung des Natriumsalzes von Vanillidenaceton: 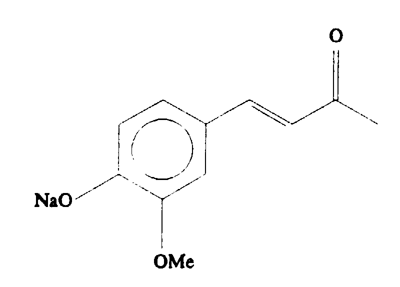 4,9 g (0,032 mol) Vanillin wurden in 15 ml Aceton gelöst und mit 5,3 ml 50%iger Natronlauge versetzt. Anschließend wurden 10 ml Wasser hinzugegeben und 20 Stunden bei Raumtemperatur gerührt. Der ausgefallene Rückstand wurde mit Aceton gewaschen und getrocknet. Man erhielt 7 g (entsprechend einer Ausbeute von 95%) der Titelverbindung in Form eines orange-gelb-gefärbten Feststoffes. Die Reinheit des Produktes betrug > 97%.

### Beispiel 2

Herstellung des Natriumsalzes von Ethyl-Vanillidenaceton: 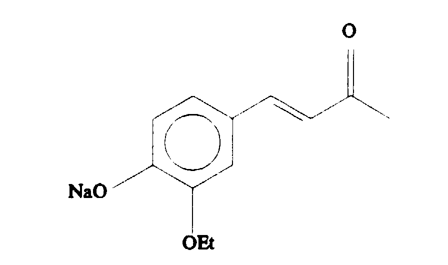 Die Titelverbindung wurde analog zu Beispiel 1 aus Ethyl-Vanillin, Natronlauge und Aceton mit einer Ausbeute von 95% hergestellt.

### Beispiel 3

Herstellung des Kaliumsalzes von 4-Hydroxybenzylidenaceton: 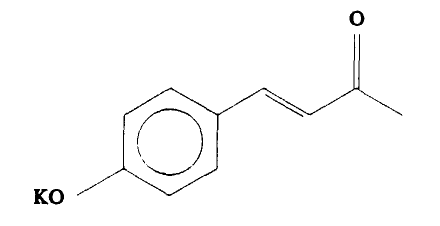 Die Titelverbindung wurde analog zu Beispiel 1 aus 4-Hydroxybenzaldehyd, Kalilauge und Aceton mit einer Ausbeute von 50% hergestellt.

### Beispiel 4

Herstellung des Kaliumsalzes von 4-Hydroxybenzalacetessigsäuremethylester: 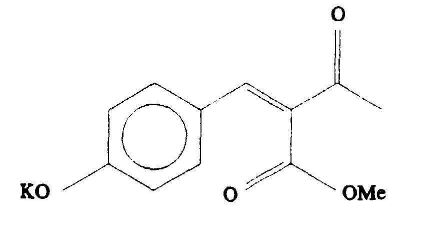 In 459 ml Methylcyclohexan wurden 232 g (2 mol) Acetessigsäuremethylester, 244 g (2 mol) 4-Hydroxybenzaldehyd, 24 g (0,4 mol) Eisessig und 6,8 g (0,08 mol) Piperidin gelöst. Die Reaktionslösung wurde auf Siedetemperatur erwärmt, und das Reaktionswasser wurde eine Stunde am Wasserabscheider ausgekreist. Nach dem Abdekantieren des Lösungsmittels wurde das Produkt in Diethylether aufgenommen, abgesaugt und getrocknet. Man erhielt 362 g (entsprechend einer Ausbeute von 82%) 4-Hydroxybenzalacetessigsäuremethylester. 22 g (0,1 mol) 4-Hydroxybenzalacetessigsäuremethylester wurden in 100 ml Methanol gelöst und mit 5,6 g (0,1 mol) KOH in 38 g Methanol versetzt. Nach 2,5-stündigem Rühren bei Raumtemperatur wurde die Reaktionsmischung eingeengt und der verbliebene Rückstand getrocknet. Man erhielt 25 g (entsprechend einer Ausbeute von 97%) der Titelverbindung in Form eines roten Feststoffes. Die Reinheit des Produktes betrug > 95%.

### Beispiel 5

Herstellung des Kaliumsalzes von 4-Hydroxybenzalacetessigsäuremethylamid: 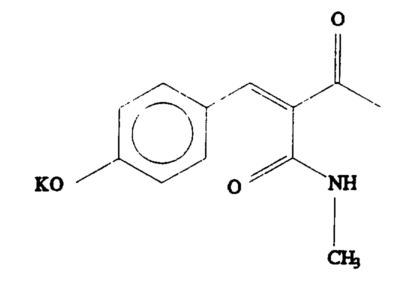 Die Titelverbindung wurde analog zu Beispiel 4 aus 4-Hydroxybenzaldehyd und Acetessigsäuremethylamid mit einer Ausbeute von 62% hergestellt.

### Beispiel 6

Herstellung des Kaliumsalzes von 4-Hydroxybenzalacetessigsäurebenzylamid: 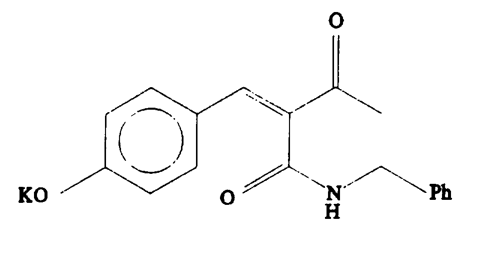 Die Titelverbindung wurde analog zu Beispiel 4 aus 4-Hydroxybenzaldehyd und Acetessigsäurebenzylamid mit einer Ausbeute von 83% hergestellt.

### Beispiel 7

Herstellung des Kaliumsalzes von 3-Hydroxybenzalacetessigsäuremethylester: 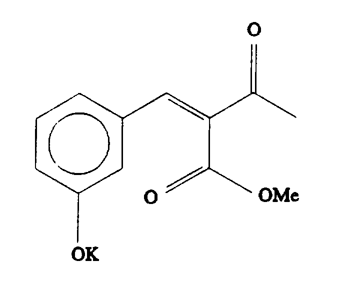 Die Titelverbindung wurde analog zu Beispiel 4 aus 4-Hydroxybenzaldehyd und Acetessigsäuremethylester mit einer Ausbeute von 43% hergestellt.

### Beispiel 8

Herstellung des Kaliumsalzes von Vanillidenacetessigsäuremethylester: 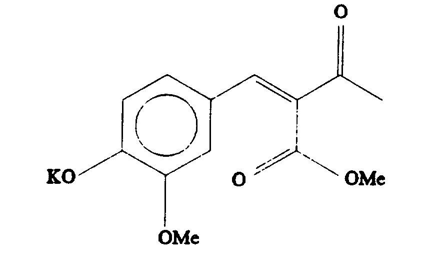 Die Titelverbindung wurde analog zu Beispiel 4 aus Vanillin und Acetessigsäuremethylester mit einer Ausbeute von 98% hergestellt.

### Beispiel 9

Herstellung des Kaliumsalzes von 4-Hydroxybenzalcyanessigsäureamid: 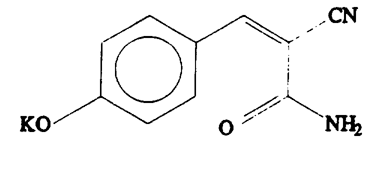 Die Titelverbindung wurde analog zu Beispiel 4 aus 4-Hydroxybenzaldehyd und Cyanessigsäureamid mit einer Ausbeute von 80% hergestellt.

### Beispiel 10

Herstellung des Kaliumsalzes von 2-Hydroxybenzalacetessigsäurebenzylamid: 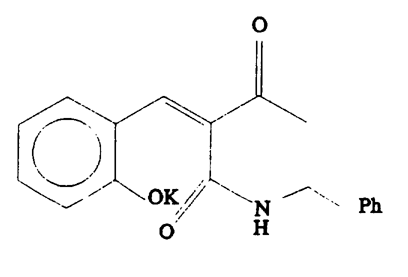 Die Titelverbindung wurde analog zu Beispiel 4 aus 4-Salicylaldehyd und Acetessigsäurebenzylamid mit einer Ausbeute von 78% hergestellt.

### Beispiel 11

Herstellung des Kaliumsalzes von 4-Hydroxybenzalacetessigsäurephenylamid: 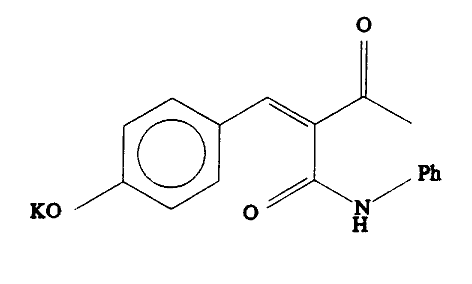 Die Titelverbindung wurde analog zu Beispiel 4 aus 4-Hydroxybenzaldehyd und Acetessigsäurephenylamid mit einer Ausbeute von 98% hergestellt.

### Beispiel 12

Herstellung des Kaliumsalzes von 3-Hydroxybenzaldimethylmalonat: 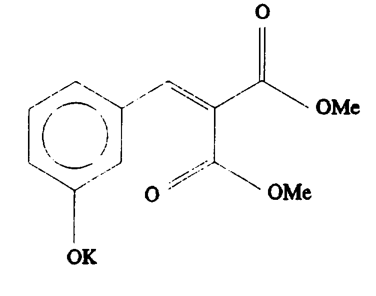 Die Titelverbindung wurde analog zu Beispiel 4 aus 3-Hydroxybenzaldehyd und Dimethylmalonat mit einer Ausbeute von 51% hergestellt.

### Beispiel 13

Herstellung des Kaliumsalzes von 7-Hydroxy-3-acetyl-cumarin: 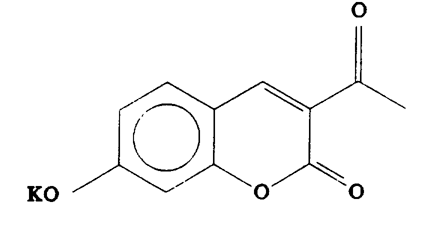 Die Titelverbindung wurde analog zu Beispiel 5 aus 2,4-Dihydroxybenzaldehyd und Acetessigsäuremethylester mit einer Ausbeute von 42% hergestellt.

### Beispiel 14

Herstellung des Kaliumsalzes von 4-Hydroxybenzalacetessigsäureethylester; 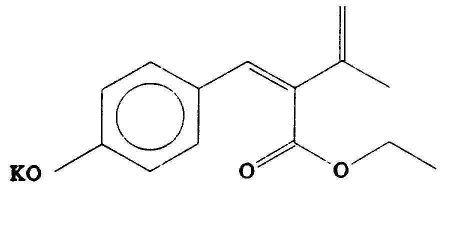 Die Titelverbindung wurde analog zu Beispiel 4 aus 4-Hydroxybenzaldehyd und Acetessigsäureethylester mit einer Ausbeute von 95% hergestellt.

### Beispiel 15

Herstellung des Kaliumsalzes von Vanillidenaceton: 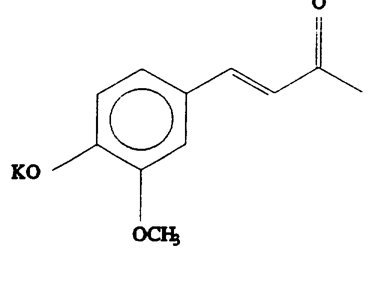 Die Titelverbindung wurde analog zu Beispiel 1 aus Vanillin, Aceton und Kaliumhydroxid mit einer Ausbeute von 92% hergestellt.

### Beispiel 16

Herstellung des Natriumsalzes von 2-Hydroxy-5-methyl-benzophenon: 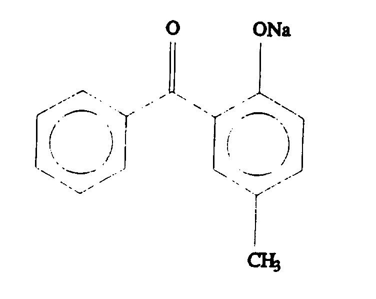 38 g (0,3 Mol) Dimethylcyclohexylamin und 27 g (0,25 Mol) p-Kresol wurden in 150 ml Chlorbenzol vorgelegt. Es wurden 35,1 g (0,25 Mol) Benzoylchlorid über einen Zeitraum von einer Stunde zugetropft, wobei die Temperatur auf 50 °C anstieg. Nach dem Abkühlen auf Raumtemperatur wurde die Suspension zur Abtrennung des Dimethylcyclohexylamin-Hydrochlorids in 100 ml Wasser gegeben. Die organische Phase wurde abgetrennt und durch Andestillieren getrocknet. Bei Raumtemperatur wurden dann innerhalb von 30 Minuten 33 g (0,25 Mol) Aluminiumtrichlorid portionsweise zugegeben. Die Innentemperatur stieg dabei auf 50 °C an. Der Ansatz wurde bis zur beendeten HCl-Ausgasung bei 130 °C ca. 10 Stunden gerührt. Nach Abkühlen auf 60 °C wurde das Reaktionsgemisch auf Eiswasser gegossen. Die Phasen wurden getrennt, und die wäßrige Phase wurde zweimal mit Chlorbenzol extrahiert. Die vereinigten organischen Phasen wurden über Magnesiumsulfat getrocknet und anschließend eingeengt. Man erhielt 43 g (entsprechend einer Ausbeute von 81 %) des 2-Hydroxy-5-Methylbenzophenons. 25 g (0,12 Mol) der Verbindung wurden dann in 150 ml Methanol gelöst und mit 4,1 g Natronlauge in 30 ml Methanol versetzt. Das Reaktionsgemisch wurde 3 Stunden bei Rückfluß gerührt und anschließend am Rotationsverdampfer eingeengt. Man erhielt 27 g (entsprechend einer Ausbeute von 98 %) der Titelverbindung in Form eines orangegefärbten Feststoffes.

### Beispiel 17

Herstellung des Natriumsalzes von 2-Hydroxy-4-methoxy-benzophenon: 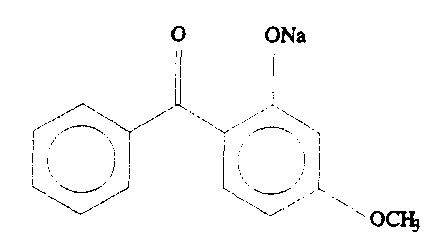 Die Titelverbindung wurde analog zu Beispiel 16 aus Benzoylchlorid und 3-Methoxyphenol mit einer Ausbeute von 70 % hergestellt.

### Beispiel 18

Herstellung des Natriumsalzes von 2-Hydroxybenzophenon: 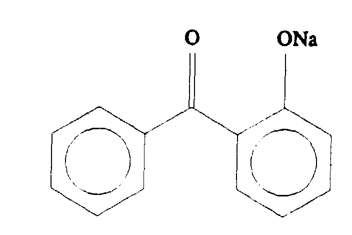 Die Titelverbindung wurde analog zu Beispiel 16 aus Benzoylchlorid und Phenol mit einer Ausbeute von 68 % hergestellt.

### Beispiel 19

Herstellung des Kaliumsalzes von 2-Hydroxy-1-naphthaldehyd: 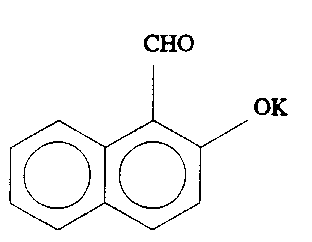 17,2 g 2-Hydroxy-1-naphthaldehyd wurden in 100 ml Methanol gelöst und mit 5,6 g KOH in 40 ml Methanol versetzt. Das Reaktionsgemisch wurde 1,5 Stunden unter Rückfluß gerührt und anschließend eingeengt. Man erhält 20 g (entsprechend einer Ausbeute von 95 %) der Titelverbindung in Form eines dunkelgrünen Feststoffes.

### Beispiel 20

Herstellung des Kaliumsalzes von 2-Hydroxy-5-methylbenzophenon: 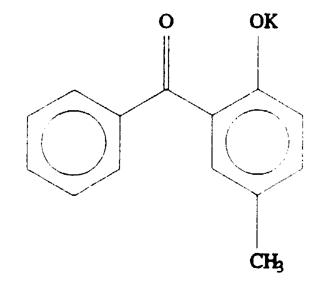 Die Titelverbindung wurde analog zu Beispiel 16 aus Benzoylchlorid, p-Kresol und Kalilauge mit einer Ausbeute von 72 % hergestellt.

### 2. Anwendungsbeispiele

Die Wirksamkeit von Glanzbildnern der Formel I bzw. II in einem Galvanisierungsverfahren wird anhand von drei verschiedenen wäßrig-sauren galvanischen Bädern demonstriert. Die erfindungsgemäß beanspruchten Glanzbildner wurden als 1 %-ige alkalische wäßrige Lösungen mit einem pH-Wert von ≥ 8, vorzugsweise von ≥ 12 (eingestellt mit NaOH) und die in den Vergleichsbeispielen angeführten Glanzbildner als 1 %-ige methanolische Lösung zuzugeben.

Ebenso können auch höher konzentrierte Lösungen eingesetzt werden.

Zusammensetzung der Bäder:

Jedes Bad enthielt, bezogen auf 1 l wäßrige Lösung:

| | |
|---|---|
| Zinkchlorid | 100 g/l |
| Kaliumchlorid | 200 g/l |
| Borsäure | 20 g/l |
| | |
| handelsübliches Naphtalinsulfonsäure/Formaldehyd-Kondensationsprodukt | 2 g/l |
| Natriumbenzoat | 2 g/l |
| handelsüblicher Fettalkoholpolyethylenglykolether mit 10 Ethylenglykol-Einheiten weiterhin: | I g/l |
| Nonylphenolpolyethylenglykolether, sulfoniert und sulfatiert, mit 10 Ethylenglykol-Einheiten | |
| **Bad 1:** | 4 g/l |
| **Bad 2:** | 10 g/l |
| **Bad 3:** | 2 g/l |

sowie erfindungsgemäßer Glanzbildner: siehe Tabelle I

Der pH-Wert der Bäder betrug 4,8. Die Einstellung des pH-Wertes erfolgte jeweils mit verdünnter Salzsäure. Die Verzinkung von Messing- oder Stahlblechen dauerte jeweils 10 Minuten. Sie wurde in einer 250 ml-Hull-Zelle mit 1 bzw. 2 Ampere bei der angegebenen Temperatur durchgeführt. Im Anschluß an die Verzinkung wurde das Blech kurz in 0,5%ige HNO₃-Lösung getaucht und dann eine Blauchromatierung vorgenommen.

Die Qualität der erhaltenen Zinküberzüge ist der folgenden Tabelle I zu entnehmen. Glanz und Duktilität der galvanischen Überzüge wurden visuell beurteilt und erhielten die Noten
1 = schlecht, 2 = gering, 3 = mäßig, 4 = gut und 5 = sehr gut.

Der Tabelle I läßt sich entnehmen, daß die Wirkung der in den galvanischen Bädern eingesetzten erfindungsgemäßen Verbindungen der Formeln I bzw. II. der Wirkung von Vergleichsverbindungen des Standes der Technik mindestens gleichkommt.

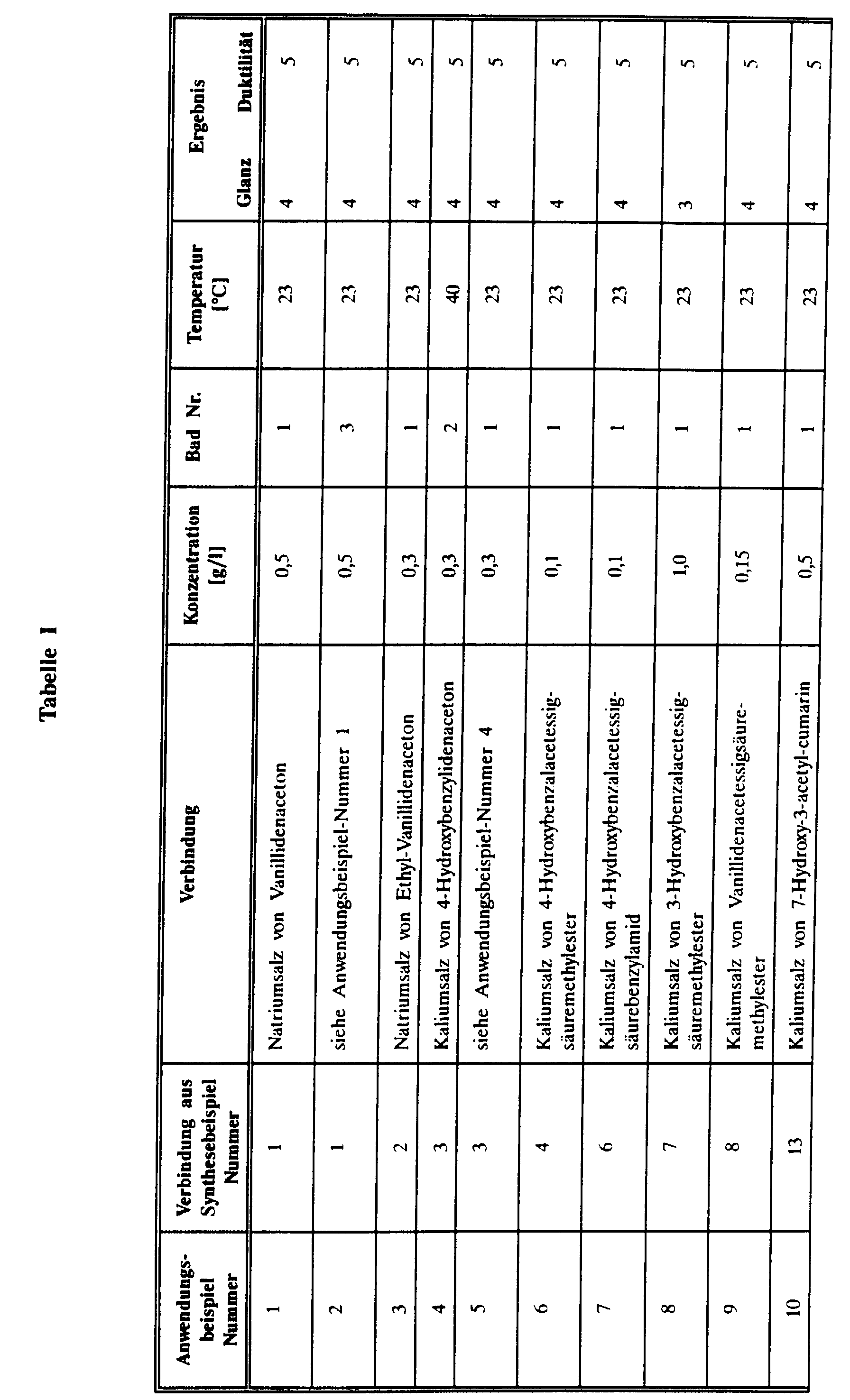

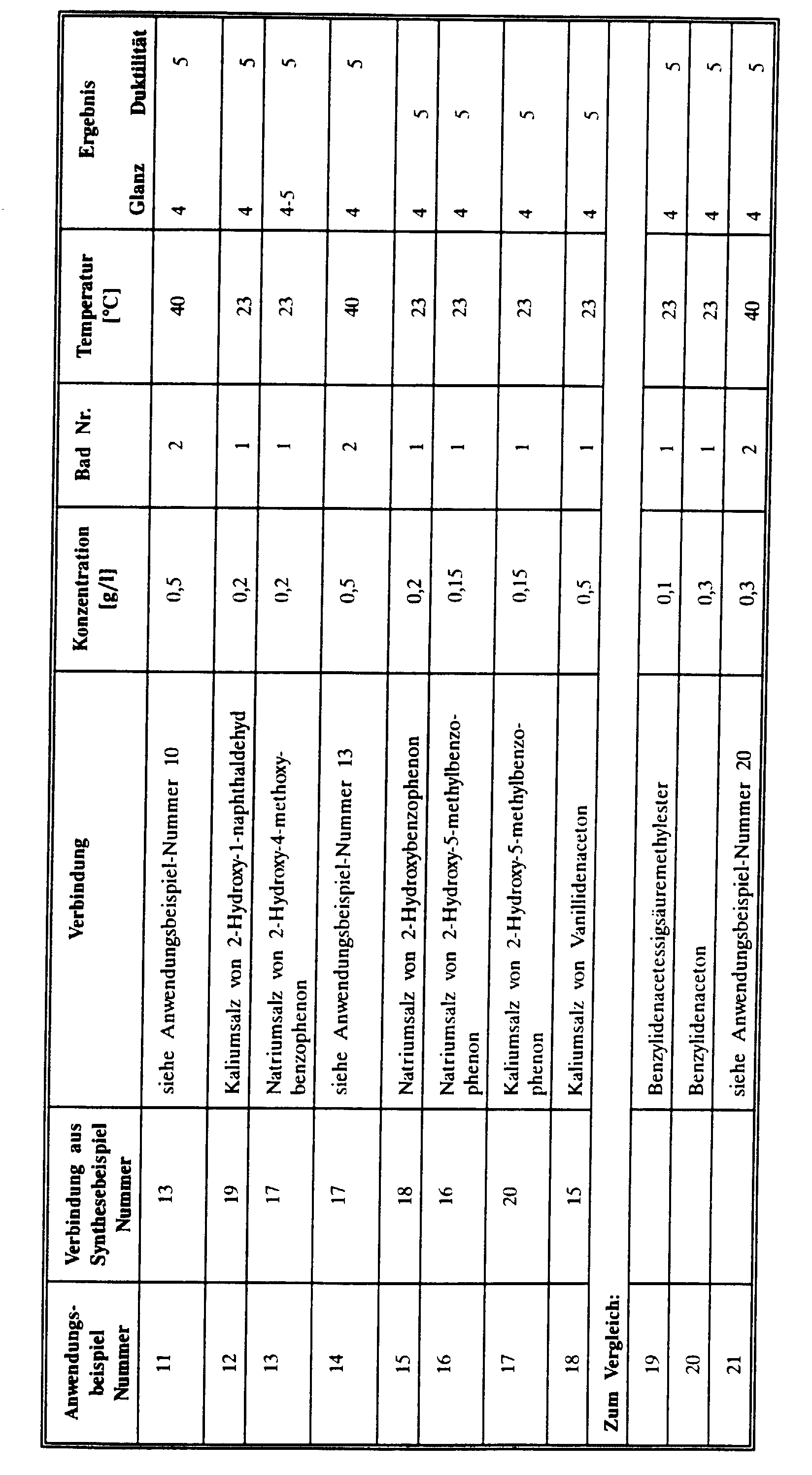

## Patentansprüche

1. Saures galvanisches Bad zur elektrolytischen Abscheidung von metallischen Schichten auf Formteilen, umfassend ein oder mehrere Metall-- salz(e), einen oder mehrere Glanzbildner, gegebenenfalls ein oder mehrere Leitsalz(e), und gegebenenfalls einen oder mehrere Hilfsglanzbildner, dadurch gekennzeichnet, daß das saure galvanische Bad als Glanzbildner mindestens eine Verbindung umfaßt, die ausgewählt ist aus den Verbindungen der Formel I 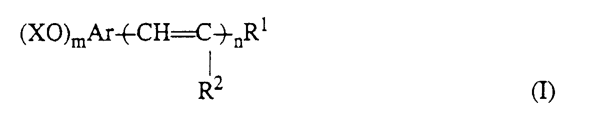 worin
m für eine ganze Zahl ≥ 1 steht;
n 0 oder 1 bedeutet;
Ar einen gegebenenfalls mit einem oder mehreren Resten, die gleich oder verschieden sein können und ausgewählt sind aus
C₁-C₈-Alkyl-,
C₁-C₈-Alkoxy-,
C₁-C₄-Alkoxycarbonyl-,
Halogen-,
Phenyl- oder
Benzylresten
substituierten Phenylen- oder Naphthylenrest bedeutet;
R¹ einen Cyano-, oder einen Acylrest der Formel -COR³ bedeutet, worin
R³ für einen gegebenenfalls mit einem oder mehreren Resten, die gleich oder verschieden sein können und ausgewählt sind aus
C₁-C₄-Alkoxycarbonyl-,
C₁-C₄-Alkoxy-
Carbonyl- oder
Cyanoresten
substituierten C₁-C₈-Alkyl-, C₂-C₈-Alkenyl- oder C₂-C₈-Alkinylrest;
für einen gegebenenfalls mit einem oder mehreren Resten, die gleich oder verschieden sein können und ausgewählt sind aus
C₁-C₄-Alkyl-,
C₁-C₄-Alkoxy-,
C₁-C₄-Alkoxycarbonyl-,
Halogen-,
Hydroxyl- oder
Phenylresten
substituierten Phenyl- oder Benzylrest;
für einen C₅-C₈-Cycloalkyirest;
für einen Hydroxylrest;
für einen C₁-C₄-Alkoxyrest; oder
für ein Wasserstoffatom steht;
R² ein Wasserstoffatom,
einen C₁-C₈-Alkyl-,
Phenyl-,
Benzyl-,
Cyano- oder
Hydroxlyrest, oder
einen Rest der Formel
-COR⁴,
-COOR⁴,
-COCH₂COOR⁴,
-OR⁴ oder
-CONR⁴R⁵,
worin
R⁴ und R⁵, die gleich oder verschieden sein können,
für ein Wasserstoffatom,
einen C₁-C₈-Alkyl-,
Phenyl- oder
Benzylrest stehen,
und
X ein Alkalimetall-,
Erdalkalimetallatom oder
einen Ammoniumrest bedeutet und
Verbindungen der Formel II 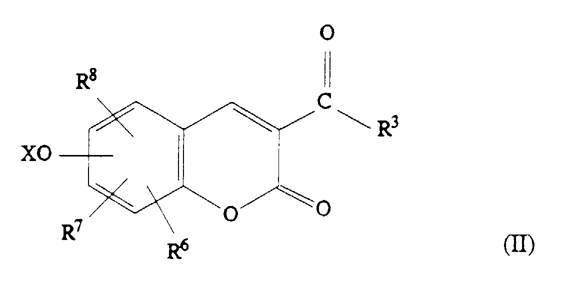 worin
R⁶, R⁷ und R⁸, die gleich oder verschieden sein können,
ein Wasserstoffatom,
einen C₁-C₈-Alkyl-,
C₁-C₈-Alkoxy-,
C₁-C₄-Alkoxycarbonyl-,
Halogen-,
Phenyl- oder
Benzylrest, oder
einen Rest der Formel -OX bedeuten, und
R³ und X die vorstehend angegebenen Bedeutungen besitzen.

2. Saures galvanisches Bad gemäß Anspruch 1,
worin in Formel I
n für 1 steht und
Ar einen gegebenenfalls mit einem oder mehreren, insbesondere mit einem oder zwei Resten, die gleich oder verschieden sein können und ausgewählt sind aus
C₁-C₄-Alkyl-, insbesondere Methyl- oder Ethyl-;
C₁-C₄-Alkoxy-, insbesondere Methoxy- oder Ethoxyresten; und
Br oder Cl;
substituierten Phenylenrest bedeutet;
und/oder
R¹ einen Cyano- oder
einen Acylrest der Formel -COR³ bedeutet,
worin
R³ für einen gegebenenfalls mit
einem oder mehreren C₁-C₄-Alkoxy-, insbesondere Methoxy- oder Ethoxyresten, die gleich oder verschieden sein können,
substituierten C₁-C₄-Alkyl-, insbesondere Methyl- oder Ethylrest;
einen gegebenenfalls mit einem oder mehreren Resten, die gleich oder verschieden sein können und ausgewählt sind aus
C₁-C₄-Alkyl-, insbesondere Methyl- oder Ethyl-;
C₁-C₄-Alkoxy-, insbesondere Methoxy- oder Ethoxy-;
Hydroxylresten; oder
Br oder Cl;
substituierten Phenyl- oder Benzylrest;
einen Hydroxylrest; oder
einen C₁-C₄-Alkoxyrest steht;
und/oder
R² ein Wasserstoffatom; einen Rest der Formel
-COR⁴;
-COOR⁴;
-COCH₂COOR⁴; oder
-CONR⁴R⁵;
worin
R⁴ und R⁵, die gleich oder verschieden sein können, für
ein Wasserstoffatom;
einen C₁-C₄-Alkyl-, insbesondere Methyl- oder Ethyl-;
einen Phenyl-; oder
einen Benzylrest stehen, bedeutet.

3. Saures galvanisches Bad gemäß Anspruch 1,
worin in Formel I
n für 0 steht und
Ar einen gegebenenfalls mit einem oder mehreren, insbesondere mit einem oder zwei Resten, die gleich oder verschieden sein können und ausgewählt sind aus
C₁-C₄-Alkyl-, insbesondere Methyl- oder Ethyl-;
C₁-C₄-Alkoxy-, insbesondere Methoxy- oder Ethoxy-;
Halogen-, insbesondere Br oder Cl; und
Hydroxylresten
substituierten Phenylen- oder Naphtylenrest bedeutet; und/oder
R¹ einen Acylrest der Formel -COR³ bedeutet,
worin
R³ für einen gegebenenfalls mit einem oder mehreren C₁-C₄-Alkoxy-, insbesondere Methoxy- oder Ethoxyresten, die gleich oder verschieden sein können, substituierten C₁-C₄-Alkylrest;
für einen gegebenenfalls mit einem oder mehreren Resten, die gleich oder verschieden sein können und ausgewählt sind unter
C₁-C₄-Alkyl-, insbesondere Methyl- oder Ethyl-;
C₁-C₄-Alkoxy-, insbesondere Methoxy- oder Ethoxy-;
Br oder Cl
substituierten Benzyl- oder Phenylrest; oder für ein Wasserstoffatom steht.

4. Saures galvanisches Bad gemäß Anspruch 1,
worin in Formel II
R³ eine der in Anspruch 2 angegebenen Bedeutungen besitzt;
und/oder
R⁶, R⁷ und R⁸, die gleich oder verschieden sein können,
ein Wasserstoffatom,
einen C₁-C₄-Alkoxy-, insbesondere Methoxy- oder Ethoxyrest, oder
einen Rest der Formel -OX bedeuten, worin
X eine der in Anspruch 1 angegebenen Bedeutungen besitzt.

5. Saures galvanisches Bad gemäß einem der Ansprüche 1 bis 4, worin X ein Alkalimetallatom, insbesondere Na oder K, bedeutet.

6. Saures galvanisches Bad gemäß Anspruch 1,
worin in Formel I
n für 1 steht;
m für 1 steht;
Ar einen gegebenenfalls mit einem
Methoxy- oder Ethoxyrest
substituierten Phenylenrest bedeutet;
R¹ einen Cyano- oder
einen Acylrest der Formel -COR³ bedeutet, worin
R³ für einen
Methyl- oder Ethyl-;
Methoxy- oder Ethoxyrest
steht; und
R² ein Wasserstoffatom,
einen Rest der Formel
-COR⁴;
-COOR⁴;
-CONHR⁵; oder
-CONR⁴R⁵ bedeutet,
worin R⁴ für einen
Methyl- oder Ethylrest steht; und
R⁵ für
ein Wasserstoffatom;
einen Methyl- oder Ethyl-;
Phenyl-; oder
Benzylrest steht;
und
X Na oder K bedeutet.

7. Saures galvanisches Bad gemäß Anspruch 1,
worin in Formel I
n für 0 steht;
m für 1 steht;
Ar einen gegebenenfalls mit einem
Methyl- oder Ethyl-;
Methoxy- oder Ethoxy-;
substituierten Phenylen- oder Naphthylenrest bedeutet;
R¹ einen Acylrest der Formel -COR³ bedeutet,
worin
R³ für ein Wasserstoffatom oder
einen Phenylrest steht;
und
X Na oder K bedeutet.

8. Saures galvanisches Bad gemäß Anspruch 1,
worin in Formel II
R³ einen Methoxy- oder Ethoxyrest bedeutet;
einer der Reste R⁶, R⁷ und R⁸
ein Wasserstoffatom; oder
einen Methoxy- oder Ethoxyrest,
und die anderen beiden Reste jeweils ein
Wasserstoffatom bedeuten; und
X Na oder K bedeutet.

9. Saures galvanisches Bad gemäß einem der Ansprüche 1 bis 8, dadurch gekennzeichnet, daß das Bad mindestens ein Metallsalz umfaßt, das ausgewählt ist aus Zink- und Zinnsalzen.

10. Saures galvanisches Bad gemäß einem der Ansprüche 1 bis 9, dadurch gekennzeichnet, daß das Bad mindestens ein Leitsalz umfaßt, das ausgewählt ist aus Natrium-, Kalium- und/oder Ammoniumsalzen.

11. Saures galvanisches Bad gemäß einem der Ansprüche 1 bis 10, dadurch gekennzeichnet, daß das Bad mindestens einen Hilfsglanzbildner umfaßt, der ein Tensid ist.

12. Saures galvanisches Bad gemäß einem der Ansprüche 1 bis 11, dadurch gekennzeichnet, daß das Bad mindestens einen zusätzlichen Glanzbildner umfaßt, der ein Benzoat, bevorzugt ein Alkalimetall- oder Ammonium. benzoat, insbesondere Natriumbenzoat ist.

13. Saures galvanisches Bad gemäß einem der Ansprüche 1 bis 12, dadurch gekennzeichnet, daß der Glanzbildner der Formel I oder II in einer Konzentration von 0,01-3 g/l, bevorzugt von 0,05-1,5 g/l, und besonders bevorzugt von 0,1-0,7 g/l, vorliegt.

14. Saures galvanisches Bad gemäß einem der Ansprüche 1 bis 13, dadurch gekennzeichnet, daß das Bad einen pH-Wert von 3 bis 7, bevorzugt von 4 bis 5, aufweist.

15. Verfahren zur galvanischen Beschichtung von Formteilen, dadurch gekennzeichnet, daß man
1. ein Formteil mit einem sauren galvanischen Bad gemäß einem der Ansprüche 1 bis 14 in Kontakt bringt, und
2. eine Galvanisierung durchführt.

16. Verwendung einer der in den Ansprüchen 1 bis 8 genannten Verbindungen der Formeln I oder II als Glanzbildner.

17. Verbindung der Formel I, wie sie in einem der Ansprüche 6 oder 7 definiert ist, mit der Maßgabe, daß das Natriumsalz von Vanillidenaceton sowie das Natriumsalz von 5-Bromvanillidenaceton ausgenommen sind.

18. Verbindung der Formel II, wie sie in einem der Ansprüche 1, 4, 5 mit Rückbezug auf Anspruch 1 oder 4, oder 8 definiert ist.

## Claims

1. An acidic electroplating bath for the electrolytic deposition of metallic layers onto shaped articles, comprising one or more metal salts, one or brighteners, if required one or more conductive salts and, if required, one or more auxiliary brighteners, wherein the acidic electroplating bath comprises, as brighteners, at least one compound which is selected from the compounds of the formula I 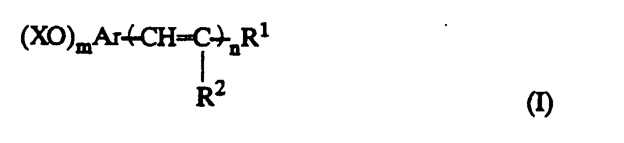 where
m is an integer ≥ 1;
n is 0 or 1;
Ar is a phenylene or naphthylene radical which is unsubstituted or substituted by one or more radicals which may be identical or different and are selected from the group consisting of
C₁-C₈-alkyl,
C₁-C₈-alkoxy,
C₁-C₄-alkoxycarbonyl,
halogen,
phenyl and
benzyl radicals;
R¹ is cyano or acyl of the formula -COR³,
where
R³ is a C₁-C₈-alkyl, C₂-C₈-alkenyl or C₂-C₈-alkynyl radical which is unsubstituted or substituted by one or more radicals which may be identical or different and are selected from the group consisting of
C₁-C₄-alkoxycarbonyl,
C₁-C₄-alkoxy,
carbonyl and
cyano radicals;
a phenyl or benzyl radical which is unsubstituted or substituted by one or more radicals which may be identical or different and are selected from the group consisting of
C₁-C₄-alkyl,
C₁-C₄-alkoxy,
C₁-C₄-alkoxycarbonyl,
halogen,
hydroxyl and
phenyl radicals;
C₅-C₈-cycloalkyl;
hydroxyl;
C₁-C₄-alkoxy or
hydrogen;
R² is hydrogen,
C₁-C₈-alkyl,
phenyl,
benzyl,
cyano or
hydroxyl, or
a radical of the formula
-COR⁴,
-COOR⁴,
-COCH₂COOR⁴,
-OR⁴ or
-CONR⁴R⁵,
where
R⁴ and R⁵, which may be identical or different, are each
hydrogen,
C₁-C₈-alkyl,
phenyl or
benzyl, and
X is an alkali metal or alkaline earth metal atom or ammonium, and
a compound of the formula II 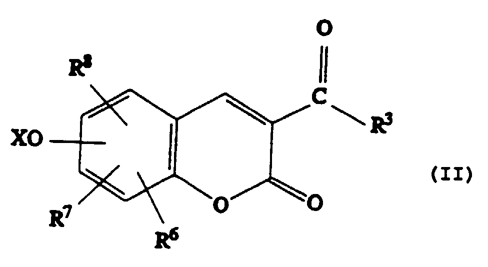 where
R⁶, R⁷ and R⁸, which may be identical or different, are each
hydrogen,
C₁-C₈-alkyl,
C₁-C₈-alkoxy,
C₁-C₄-alkoxycarbonyl,
halogen,
phenyl or
benzyl, or
a radical of the formula -OX, and
R³ and X have the meanings stated above.

2. An acidic electroplating bath as claimed in claim 1,
wherein in formula I
n is 1 and
Ar is phenylene which is unsubstituted or substituted by one or more, in particular one or two, radicals which may be identical or different and are selected from the group consisting of
C₁-C₄-alkyl, in particular methyl or ethyl;
C₁-C₄-alkoxy, in particular methoxy or ethoxy, and
Br or Cl;
or
R¹ is cyano or
acyl of the formula -COR³,
where
R³ is C₁-C₄-alkyl, in particular methyl or ethyl, which is unsubstituted or substituted by one or more C₁-C₄-alkoxy, in particular methoxy or ethoxy, radicals which may be identical or different;
a phenyl or benzyl radical which is unsubstituted or substituted by one or more radicals which may be identical or different and are selected from the group consisting of
C₁-C₄-alkyl, in particular methyl or ethyl;
C₁-C₄-alkoxy, in particular methoxy or ethoxy;
hydroxyl or
Br or Cl;
hydroxyl; or
C₁-C₄-alkoxy;
or
R² is hydrogen; a radical of the formula -COR⁴;
-COOR⁴;
-COCH₂COOR⁴ or
-CONR⁴R⁵,
where
R⁴ and R⁵, which may be identical or different, are each
hydrogen;
C₁-C₄-alkyl, in particular methyl or ethyl;
phenyl or
benzyl.

3. An acidic electroplating bath as claimed in claim 1,
wherein in formula I
n is 0 and
Ar is a phenylene or naphthylene radical which is unsubstituted or substituted by one or more, in particular one or two, radicals which may be identical or different and are selected from the group consisting of
C₁-C₄-alkyl, in particular methyl or ethyl;
C₁-C₄-alkoxy, in particular methoxy or ethoxy;
halogen, in particular Br or Cl, and
hydroxyl;
or
R¹ is acyl of the formula -COR³,
where
R³ is C₁-C₄-alkyl which is unsubstituted or substituted by one or more C₁-C₄-alkoxy, in particular methoxy or ethoxy, radicals which may be identical or different;
a benzyl or phenyl radical which is unsubstituted or substituted by one or more radicals which may be identical or different and are selected from the group consisting of
C₁-C₄-alkyl, in particular methyl or ethyl;
C₁-C₄-alkoxy, in particular methoxy or ethoxy, or
Br or Cl;
or
hydrogen.

4. An acidic electroplating bath as claimed in claim 1,
wherein in formula II
R³ has one of the meanings stated in claim 2 or
R⁶, R⁷ and R⁸, which may be identical or different, are each
hydrogen,
C₁-C₄-alkoxy, in particular methoxy or ethoxy, or a radical of the formula -OX, where
X has one of the meanings stated in claim 1.

5. An acidic electroplating bath as claimed in any of claims 1 to 4, wherein
X is an alkali metal atom, in particular Na or K.

6. An acidic electroplating bath as claimed in claim 1,
wherein in formula I
n is 1;
m is 1
Ar is phenylene which is unsubstituted or substituted by methoxy or ethoxy;
R¹ is cyano or acyl of the formula -COR³, where
R³ is
methyl or ethyl;
methoxy or ethoxy;
and
R² is hydrogen;
a radical of the formula
-COR⁴;
-COOR⁴;
-CONHR⁵ or
-CONR⁴R⁵,
where R⁴ is
methyl or ethyl; and
R⁵ is
hydrogen;
methyl or ethyl;
phenyl or
benzyl;
and
X is Na or K.

7. An acidic electroplating bath as claimed in claim 1,
wherein in formula I
n is 0;
m is 1;
Ar is a phenylene or naphthylene radical which is unsubstituted or substituted by
methyl or ethyl;
methoxy or ethoxy;
R¹ is an acyl of the formula -COR³,
where
R³ is hydrogen or
phenyl
and
X is Na or K.

8. An acidic electroplating bath as claimed in claim 1,
wherein in formula II
R³ is methoxy or ethoxy and one of the radicals R⁶, R⁷ and R⁸ is
hydrogen or
methoxy or ethoxy and the other two radicals are each hydrogen; and
X is Na or K.

9. An acidic electroplating bath as claimed in any of claims 1 to 8, wherein the bath comprises at least one metal salt which is selected from zinc salts and tin salts.

10. An acidic electroplating bath as claimed in any of claims 1 to 9, wherein the bath comprises at least one conductive salt which is selected from sodium salts, potassium salts or ammonium salts or from sodium salts, potassium salts and ammonium salts.

11. An acidic electroplating bath as claimed in any of claims 1 to 10, wherein the bath comprises at least one auxiliary brightener which is a surfactant.

12. An acidic electroplating bath as claimed in any of claims I to 11, wherein the bath comprises at least one additional brightener which is a benzoate, preferably an alkali metal or ammonium benzoate, in particular sodium benzoate.

13. An acidic electroplating bath as claimed in any of claims 1 to 12, wherein the brightener of the formula I or II is present in a concentration of 0.01-3, preferably 0.05-1.5, particularly preferably 0.1-0.7, g/l.

14. An acidic electroplating bath as claimed in any of claims 1 to 13, wherein the bath has a pH of from 3 to 7, preferably from 4 to 5.

15. A process for the electroplating of shaped articles, which comprises
1. bringing a shaped article into contact with an acidic electroplating bath as claimed in any of claims 1 to 14 and
2. carrying out electroplating.

16. The use of one of the compounds of the formula I or II which are stated in any of claims I to 8 as brighteners.

17. A compound of the formula I as defined in claim 6 or 7, with the proviso that the sodium salt of vanillideneacetone and the sodium salt of 5-bromovanillideneacetone are excluded.

18. A compound of the the formula II as defined in claim 1, 4, 5 (referring back to claim 1 or 4), or 8.

## Revendications

1. Bain électrolytique acide pour la déposition électrolytique de couches métalliques sur des objets façonnés, comprenant un ou plusieurs sels métalliques, un ou plusieurs agents de lustrage, éventuellement un ou plusieurs sels conducteurs, et éventuellement un ou plusieurs agents de lustrage auxiliaires, caractérisé en ce que le bain électrolytique acide contient en tant qu'agents de lustrage au moins un composé qui est choisi parmi les composés de formule I 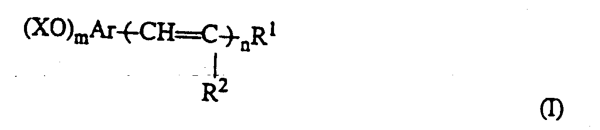 dans laquelle
m est un nombre entier ≥ 1 ;
n vaut 0 ou 1 ;
Ar est un radical phénylène ou naphtylène, éventuellement substitué par un ou plusieurs radicaux qui peuvent être identiques ou différents et qui sont choisis parmi les radicaux alkyle en C₁-C₈, alcoxy en C₁-C₈, alcoxy-carbonyle en C₁-C₄, halogéno, phényle ou benzyle ;
R¹ est un radical cyano ou un radical acyle de formule -COR³, dans laquelle R³ est un radical alkyle en C₁-C₈, alcényle en C₂-C₈, ou alcynyle en C₂-C₈, éventuellement substitué par un ou plusieurs radicaux, qui peuvent être identiques ou différents et qui sont choisis parmi les radicaux alcoxy-carbonyle en C₁-C₄, alcoxy en C₁-C₄, carbonyle ou cyano ; un radical phényle ou benzyle éventuellement substitué par un ou plusieurs radicaux qui peuvent être identiques ou différents, et qui sont choisis parmi les radicaux alkyle en C₁-C₄, alcoxy en C₁-C₄, alcoxycarbonyle en C₁-C₄, halogéno, hydroxyle ou phényle ; un radical cycloalkyle en C₅-C₈, un radical hydroxyle ; un radical alcoxy en C₁-C₄ ; ou un atome d'hydrogène ;
R² est un atome d'hydrogène, un radical alkyle en C₁-C₈, phényle, benzyle, cyano ou hydroxyle, ou encore un radical de formule -COR⁴, -COOR⁴, -COCH₂COOR⁴, -OR⁴ ou -CONR⁴R⁵, où R⁴ et R⁵, qui peuvent être identiques ou différents, représentent chacun un atome d'hydrogène ou un radical alkyle en C₁-C₈, phényle ou benzyle; et
X est un atome d'un métal alcalin, un atome d'un métal alcalino-terreux ou un radical ammonium, et les composés de formule II 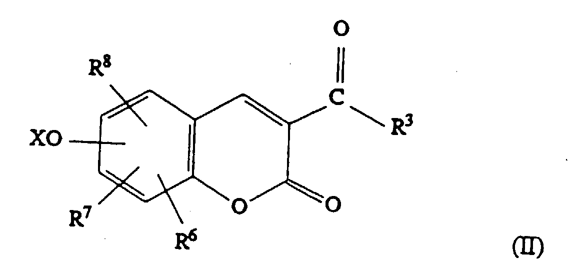 dans laquelle
R⁶, R⁷ et R⁸, qui peuvent être identiques ou différents, représentent chacun un atome d'hydrogène, un radical alkyle en C₁-C₈, alcoxy en C₁-C₈, alcoxycarbonyle en C₁-C₄, halogéno, phényle ou benzyle, ou encore un radical de formule -OX, et
R³ et X ont les significations données ci-dessus.

2. Bain électrolytique acide selon la revendication 1, dans lequel, dans la formule I :
n vaut 1, et
Ar est un radical phénylène éventuellement substitué par un ou plusieurs et en particulier par un ou deux radicaux, qui peuvent être identiques ou différents, et qui sont choisis parmi les radicaux alkyle en C₁-C₄, en particulier méthyle ou éthyle, alcoxy en C₁-C₄, en particulier méthoxy ou éthoxy, et Br ou Cl ;
et/ou
R¹ est un radical cyano ou un radical acyle de formule -COR³, dans laquelle R³ est un radical alkyle en C₁-C₄, en particulier méthyle ou éthyle, éventuellement substitué par un ou plusieurs substituants alcoxy en C₁-C₄, en particulier méthoxy ou éthoxy, qui peuvent être identiques ou différents ; un radical phényle ou benzyle, éventuellement substitué par un ou plusieurs radicaux qui peuvent être identiques ou différents et qui sont choisis parmi les radicaux alkyle en C₁-C₄, en particulier méthyle ou éthyle, alcoxy en C₁-C₄, en particulier méthoxy ou éthoxy, hydroxyle, ou encore Br ou Cl ; un radical hydroxyle ; ou un radical alcoxy en C₁-C₄ ;
et/ou
R² est un atome d'hydrogène ; un radical de formule -COR⁴, -COOR⁴, -COCH₂COOR⁴ ou -CONR⁴R⁵, où R⁴ et R⁵, qui peuvent être identiques ou différents, représentent chacun un atome d'hydrogène, un radical alkyle en C₁-C₄, en particulier méthyle ou éthyle, un radical phényle ou un radical benzyle.

3. Bain électrolytique acide selon la revendication 1, dans lequel, dans la formule I :
n vaut 0, et
Ar est un radical phénylène ou naphtylène éventuellement substitué par un ou plusieurs et en particulier par un ou deux radicaux qui peuvent être identiques ou différents et qui sont choisis parmi les radicaux alkyle en C₁-C₄, en particulier méthyle ou éthyle, alcoxy en C₁-C₄, en particulier méthoxy ou éthoxy, halogéno, en particulier Br ou Cl, et hydroxyle ;
et/ou
R¹ est un radical acyle de formule -COR³, dans laquelle R³ est un radical alkyle en C₁-C₄ éventuellement substitué par un ou plusieurs radicaux alcoxy en C₁-C₄, en particulier méthoxy ou éthoxy, qui peuvent être identiques ou différents, un radical benzyle ou phényle éventuellement substitué par un ou plusieurs radicaux qui peuvent être identiques ou différents et qui sont choisis parmi les radicaux alkyle en C₁-C₄, en particulier méthyle ou éthyle, alcoxy en C₁-C₄, en particulier méthoxy ou éthoxy, Br ou Cl ; ou encore un atome d'hydrogène.

4. Bain électrolytique acide selon la revendication 1, dans lequel, dans la formule II :
R³ a l'une des significations données dans la revendication 2;
et/ou
R⁶, R⁷ et R⁸, qui peuvent être identiques ou différents, représentent chacun un atome d'hydrogène, un radical alcoxy en C₁-C₄, en particulier méthoxy ou éthoxy, ou un radical de formule -OX dans laquelle X a l'une des significations données dans la revendication 1.

5. Bain électrolytique acide selon l'une des revendications 1 à 4, dans lequel X est un atome d'un métal alcalin, en particulier Na ou K.

6. Bain électrolytique acide selon la revendication 1, dans lequel, dans la formule 1 :
n vaut 1 ;
m vaut 1;
Ar est un radical phénylène éventuellement substitué par un radical méthoxy ou éthoxy ;
R¹ est un radical cyano ou un radical acyle de formule -COR³ dans laquelle R³ est un radical méthyle ou éthyle, méthoxy ou éthoxy ; et
R² est un atome d'hydrogène, un radical de formule -COR⁴, -COOR⁴, -CONHR⁵ ou -CONR⁴R⁵, où R⁴ est un radical méthyle ou éthyle, et R⁵ est un atome d'hydrogène, un radical méthyle ou éthyle, phényle, ou benzyle ;
et
X est Na ou K.

7. Bain électrolytique acide selon la revendication 1, dans lequel, dans la formule I :
n vaut 0 ;
m vaut 1;
Ar est un radical phénylène ou naphtylène éventuellement substitué par un substituant méthyle ou éthyle, méthoxy ou éthoxy ;
R¹ est un radical acyle de formule -COR³, dans laquelle R³ est un atome d'hydrogène ou un radical phényle ;
et
X est Na ou K.

8. Bain électrolytique acide selon la revendication 1, dans lequel, dans la formule II :
R³ est un radical méthoxy ou éthoxy ;
l'un des radicaux R⁶, R⁷ et R⁸ est un atome d'hydrogène ou un radical méthoxy ou éthoxy, et les deux autres radicaux représentent chacun un atome d'hydrogène ; et
X est Na ou K.

9. Bain électrolytique acide selon l'une des revendications 1 à 8, caractérisé en ce que le bain contient au moins un sel métallique choisi parmi les sels de zinc et d'étain.

10. Bain électrolytique acide selon l'une des revendications 1 à 9, caractérisé en ce que le bain contient au moins un sel conducteur choisi parmi les sels de sodium, de potassium et/ou d'ammonium.

11. Bain électrolytique acide selon l'une des revendications 1 à 10, caractérisé en ce que le bain contient au moins un agent de lustrage auxiliaire, qui est un tensioactif.

12. Bain électrolytique acide selon l'une des revendications 1 à 11, caractérisé en ce que le bain contient au moins un agent de lustrage supplémentaire, qui est un benzoate, en particulier un benzoate d'un métal alcalin ou d'ammonium, en particulier le benzoate de sodium.

13. Bain électrolytique acide selon l'une des revendications 1 à 12, caractérisé en ce que l'agent de lustrage de formule I ou II est présent à une concentration de 0,01-3, de préférence de 0,05-1,5 et en particulier de 0,1-0,7 g/l.

14. Bain électrolytique acide selon l'une des revendications 1 à 13, caractérisé en ce que le bain a un pH de 3 à 7, de préférence de 4 à 5.

15. Procédé de revêtement électrolytique d'objets façonnés, caractérisé en ce que
1. on met en contact un objet façonné avec un bain électrolytique acide selon l'une des revendications 1 à 14, et
2. on procède à une déposition électrolytique.

16. Utilisation d'un des composés selon les revendications 1 à 8, ayant les formules I ou II, en tant qu'agents de lustrage.

17. Composé de formule I, tel que défini dans l'une des revendications 6 ou 7, à la condition que soient exclus le sel de sodium de la vanillidène-acétone, ainsi que le sel de sodium de la 5-bromovanillidène-acétone.

18. Composé de formule II, tel que défini dans l'une des revendications 1, 4, 5, avec référence à la revendication 1 ou 4, ou 8.
